# EUROPEAN PATENT APPLICATION

(11) **EP 3 447 657 A1**
(43) Date of publication of application: **27.02.2019**
(21) Application number: 16890127.0
(22) Date of filing: 06.12.2016
(51) Int. Cl.: G06F 17/30, G06K 9/00

(54) **SOCIAL INFORMATION SEARCH SYSTEM AND SEARCH METHOD THEREOF**

(30) Priority: 20.04.2016 CN 201610250884
(71) Applicant: Huizhou TCL Mobile Communication Co., Ltd., Huizhou, Guangdong 516006 (CN)
(72) Inventor: WANG, Song, Huizhou Guangdong 516006 (CN)
(74) Representative: ZHAOffice SPRL
(86) International application number: PCT/CN2016/108663
(87) International publication number: WO 2017/181689

(57) **Abstract**

The disclosure provides a social information search system and a method thereof. The system includes a mobile electronic device, an intelligent terminal, and a server terminal. The server terminal matches social information matching personal information of a user in a network social information database and feeds the social information to the intelligent terminal, where the personal information includes objective biological data of the user which is obtained by the mobile electronic device and subjective data input by the user which is obtained by the intelligent terminal.

## Description

### TECHNICAL FIELD

Embodiments of the present disclosure generally relate to communication technology, and in particular relate to a social information search system and method thereof.

### BACKGROUND

With the development of science and technology, there are various kinds of wearable devices. Such hardware devices realize powerful functions via software supports, data exchanges, and cloud exchanges, which bring a great change to our live. However, most social products such as microblog, WeChat, and Facebook which are popular in the current market are software-based.

In human beings' social relations, only people who have similar personality, common feature, or similar characteristic incline to get together. Accordingly, the present disclosure provides a social information search system, which combines hardware and software to recommend different users with social information which matches their character, hobby, physical condition or sports condition, thereby greatly improving the user experience.

### SUMMARY

The present disclosure provides a social information search system and a method thereof, which recommends different users with social information which matches personal information of a user according to the personal situation of the user, thereby improving the user experience.

A first aspect of the present disclosure is to provide a social information search system. The system includes: a mobile electronic device, an intelligent terminal, and a server terminal. The mobile electronic device is for wearing by a user, which includes a first bus as well as a sensor assembly, a first storage, a first processor, and a first communication interface respectively connected with the first bus; the intelligent terminal includes a second bus as well as a second storage, a second processor, a second communication interface, and a second display respectively connected with the second bus; the server terminal includes a third bus as well as a third storage, a third processor, and a third communication interface respectively connected with the third bus. The first storage stores a first program instruction, the first processor is configured to execute the first program instruction, the second storage stores a second program instruction, the second processor is configured to execute the second program instruction, the third storage stores a third program instruction , the third processor is configured to execute the third program instruction, a data transmission between any two of the first communication interface, the second communication interface, and the third communication interface is performed via Bluetooth, infrared, WiFi, Zigbee, or FM. The first processor executes the first program instruction to control the sensor assembly to detect objective biological data of the user, where the objective biological data includes status information of the user and environment information, the status information includes a heart rate, a body surface temperature, a movement trajectory, and a velocity of the user, the environment information includes a location, an atmospheric pressure, and a humidity. The first communication interface transmits the objective biological data. The second processor executes the second program instruction to control the second communication interface to receive the objective biological data, obtain subjective data input by the user, integrate the objective biological data and the subjective data into a data packet, and control the second communication interface to transmit the data packet. The third processor executes the third program instruction to control the third communication interface to receive the data packet, determines personal information of the user basing on the data packet, and search social information which matches the personal information in a network social information database. The third communication interface feeds the social information to the intelligent terminal.

In one embodiment, the subjective data includes a height, a weight, an occupation, an interest, and a hobby of the user.

In one embodiment, the mobile electronic device is a bracelet.

A second aspect of the present disclosure is to provide a social information search system. The system includes:
a mobile electronic device for wearing by a user which includes an objective data recording module configured to detect objective biological data of the user and a first communication unit configured to transmit the objective biological data; an intelligent terminal which includes a second communication unit configured to receive the objective biological data, a subjective data recording module configured to obtain subjective data input by the user, and a first processing chip configured to integrate the subjective data and the objective biological data into a data packet, while the second communication unit is further configured to transmit the data packet; a server terminal which includes a third communication unit configured to receive the data packet as well as a second processing chip configured to determine personal information of the user basing on the data packet and search social information which matches the personal information in a network social information database, while the third communication unit is further configured to feed the social information to the intelligent terminal.

In one embodiment, the objective biological data includes status information of the user and environment information, the status information includes a heart rate, a body surface temperature, a movement trajectory, and a velocity of the user, the environment information includes a location, an atmospheric pressure, and a humidity.

In one embodiment, the subjective data includes a height, a weight, an occupation, an interest, and a hobby of the user.

In one embodiment, a data transmission between any two of the first communication unit, the second communication unit, and the third communication unit is performed via Bluetooth, infrared, WiFi, Zigbee, or FM.

In one embodiment, the mobile electronic device is a bracelet.

A third aspect of the present disclosure is to provide a social information search method, including: receiving data packet which includes subjective data and objective biological data of a user from an intelligent terminal by a third communication unit; determining personal information of the user basing on the subjective data and the objective biological data and search social information which matches the personal information in a network social information database by a second processing chip; and feeding the social information to the intelligent terminal by a third communication unit.

In one embodiment, the objective biological data includes status information of the user and environment information, the status information includes a heart rate, a body surface temperature, a movement trajectory, and a velocity of the user, the environment information includes a location, an atmospheric pressure, and a humidity.

In one embodiment, the subjective data includes a height, a weight, an occupation, an interest, and a hobby of the user.

In one embodiment, the receiving the data packet including the subjective data of the user and the objective biological data from the intelligent terminal by the third communication unit includes: receiving the data packet from the intelligent terminal by the third communication unit via Bluetooth, infrared, WiFi, Zigbee, or FM.

In one embodiment, before the receiving the data packet including the subjective data of the user and the objective biological data from the intelligent terminal by the third communication unit includes: receiving the subjective data of the user from the mobile electronic device by the intelligent terminal.

Through the above-mentioned schemes, the present disclosure provides the advantages: different from the prior art, the social information search system of the present disclosure obtains the objective biological data of the user from the mobile electronic device which can be worn by the user, transmits the objective biological data which is combined with the subjective data inputted to the intelligent terminal by the user to the server terminal to match, so as to match the social information which matches the personal information in the network social information database and push the social information to the user, thereby improving the user experience.

### BRIEF DESCRIPTION OF THE DRAWINGS

FIG. 1 is a schematic diagram of a social information search system according to an embodiment of the present disclosure.
FIG. 2 is a flow chart of a social information search method based on the social information search system of FIG. 1.
FIG. 3 is a schematic diagram of the hardware of a social information search system according to an embodiment of the present disclosure.

### DETAILED DESCRIPTION

The present disclosure will be described in further detail with reference to the accompanying drawings and embodiments, in which the technical problems to be solved, the technical solutions, and the advantages of the present disclosure will become more apparent. It is to be understood that the specific embodiments described herein are merely for explaining the present disclosure, and are not intended to limit the present disclosure.

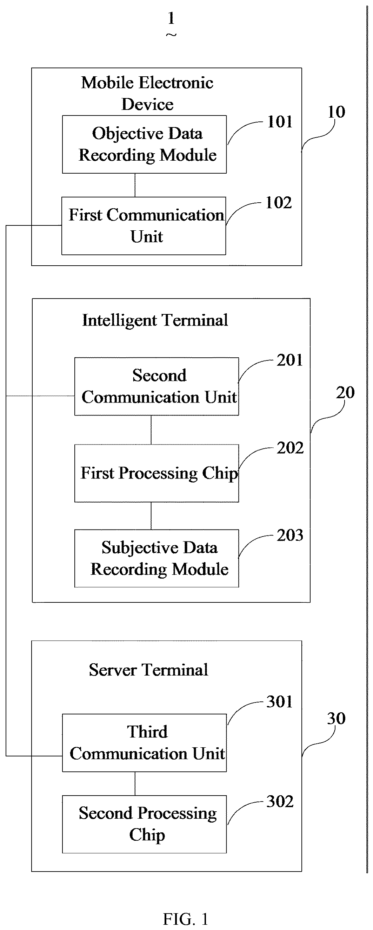

Referring to FIG. 1, a schematic diagram of a social information search system according to an embodiment of the present disclosure is depicted. As shown in FIG. 1, in this embodiment, a social information search system 1 includes a mobile electronic device 10, an intelligent terminal 20, and a server terminal 30, which utilizes the mobile electronic device 10 which can be worn by a user to obtain objective biological data of the user and the intelligent terminal 20 to obtain subjective data input by the user, so as to realize searching social information which matches personal information of the user in a cloud database and push the social information to the user. Through combining hardware and software, the user is allowed to search nearby persons who have similar interest, hobby, or common character with himself anytime and anywhere so as to make friends, which makes people's social activities more colorful.

In this embodiment, the mobile electronic device 10 is an independent electronic device with communication and computing capabilities, which can be worn on the body of the user. The mobile electronic device 10 can include but not limited to a wearable device, a mobile phone accessory, a computer accessory, a subsidiary device connected to the intelligent terminal 20, or a general or special small sensing devices with communication capability.

The intelligent terminal 20 is a terminal device having communication and computing capabilities, which can include but not limited to a mobile phone, a tablet, a laptop, and a personal electronic device with similar functions.

In this embodiment, it is preferable that the mobile electronic device 10 is a device can be worn on the body of the user such as a bracelet or a wristband, the intelligent terminal 20 is a smart phone, and the server terminal 30 is a cloud server.

In this embodiment, information transmissions between any two of the mobile electronic device 10, the intelligent terminal 20, and the server terminal 30 is performed by a local area network or a wide area network based on TCP / IP (Transmission Control Protocol / Internet Protocol). The transmission can be performed via but not limited to Bluetooth, infrared, WiFi, Zigbee, FM (Frequency Modulation), or other wireless communication manners.

The mobile electronic device 10 includes an objective data recording module 101 and a first communication unit 102. The objective data recording module 101 is configured to detect the objective biological data of the user, which can include but not limited to one or any combination of a three-dimensional motion sensor, a GPS (Global Positioning System), a humidity sensor, a temperature sensor, an electrocardiogram sensor, and an electroencephalogram sensor. The first communication unit 102 is configured to transmit the objective biological data to the intelligent terminal 20. The objective biological data includes status information of the user and environment information, where the status information can include but not limited to a heart rate, a body surface temperature, a movement trajectory, and a velocity of the user, and the environment information can include a location, an atmospheric pressure, a temperature, and a humidity, etc.

The intelligent terminal 20 includes a second communication unit 201, a first processing chip 202, and a subjective data recording module 203. The subjective data recording module 203 is configured to receive subjective data inputted by the user. The subjective data can include but not limited to a height, a weight, an occupation, an interest, and a hobby of the user. The second communication unit 201 is configured to receive the objective biological data transmitted by the mobile electronic device 10, where the reception can be performed via but not limited to Bluetooth, infrared, WiFi, Zigbee, or FM. The second processing chip 302 is configured to integrate the objective biological data and the subjective data into a data packet, and transmit the data packet to the server terminal 30 via the second communication unit 201.

The server terminal 30 includes a third communication unit 301 and a second processing chip 302. The third communication unit 301 is configured to receive the data packet. Data transmissions between the third communication unit 301 and the second communication unit 201 are performed via Bluetooth, infrared, WiFi, Zigbee, or FM. The second processing chip 302 decodes and analyzes the data packet to obtain the personal information of the user, and then searches the social information which matches the personal information of the user in a network social information database. The third communication unit 301 feeds the obtained social information to the intelligent terminal 20, so as to push the social information to the user.

In summary, this embodiment utilizes the mobile electronic device 10 worn on the body of the user to obtain the objective biological data of the user in real time, and utilizes the intelligent terminal 20 to obtain the subjective data inputted by the user, integrate the objective biological data and the subjective data to form the personal information of the user, so as to search the social information which matches the personal information in the network social information database of the cloud server and push the social information to the user, so that the user can obtain the social information of persons who have similar personality characteristic and common interest with himself anytime and anywhere, which provides much conveniences for the user to expend his social circle, and the user experience is improved.

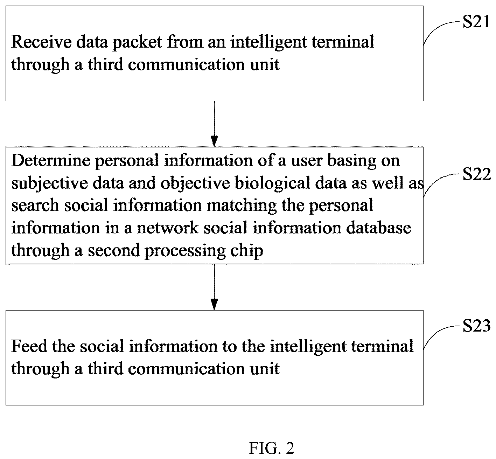

Referring to FIG. 2, a flow chart of a social information search method based on the social information search system of FIG. 1 is depicted. As shown in FIG. 2, the method can include the following blocks.

At S21: receiving the data packet from the intelligent terminal 20 through the third communication unit 301.

The data packet includes the subjective data and the objective biological data of the user. The subjective data includes a height, a weight, an occupation, an interest, and a hobby of the user, etc. The objective biological data includes status information of the user and environment information, the status information can include but not limited to a heart rate, a body surface temperature, a movement trajectory, and a velocity of the user, and the environment information can include but not limited to a location, an atmospheric pressure, and a humidity.

In this embodiment, the subjective data is obtained from the mobile electronic device 10 through the intelligent terminal 20.

At S22: determining the personal information of the user basing on the subjective data and the objective biological data and search the social information which matches the personal information in the network social information database through the second processing chip 302.

The second processing chip 302 analyzes the obtained subjective data and objective data so as to obtain the personal information of the user and searches the social information which matches the personal information in the network social information database of the cloud server based on the personal information of the user.

At S23: feeding the social information to the intelligent terminal 20 through the third communication unit 301.

The second communication unit 201 pushes the obtained social information to the user of the intelligent terminal 20.

The scene to implement the present disclosure is as follows.

The mobile electronic device 10 is a bracelet, the intelligent terminal 20 is a smart phone, and the server terminal 30 is a cloud server. There are multiple users A, B, C, and D, in which A wears a bracelet 1 and holds a smart phone 1, B wears a bracelet 2 and holds a smart phone 2, C wears bracelet 3 and holds a smart phone 3, D wears a bracelet 4 and holds a smart phone 4. In this case, A, B, C, and D respectively uploads his own subjective data to the cloud server, and uploads the objective biological data obtained by the bracelet to the cloud server. When the cloud server matches A and C who have a common hobby, the social information of A is pushed to C, and the social information of C is pushed to A, so that A and C respectively obtains the social information of each other and a dating or meeting between them can be made.

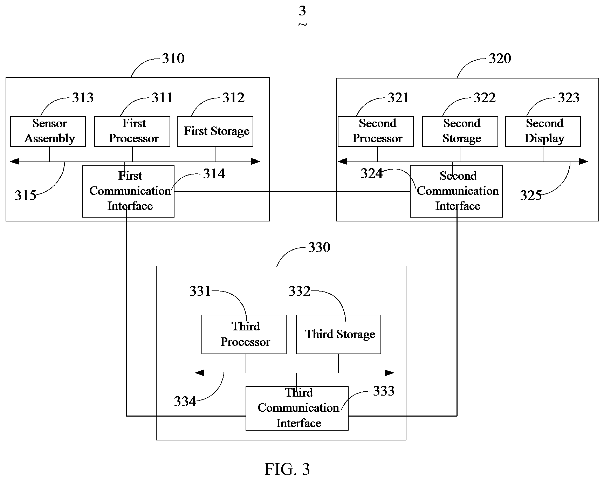

Referring to FIG. 3, a schematic diagram of the hardware of a social information search system according to an embodiment of the present disclosure is depicted. As shown in FIG. 3, the social information search system 3 includes: a mobile electronic device 310, an intelligent terminal 320, and a server terminal 330. The mobile electronic device 310 can be worn on the body of the user, which includes a first bus 315, a sensor assembly 313, a first storage 312, a first processor 311, and a first communication interface 314. The sensor assembly 313, the first storage 312, the first processor 311, and the first communication interface 314 are respectively connected with the first bus 315. The intelligent terminal 320 includes a second bus 325, a second storage 322, a second processor 321, a second communication interface 324, and a second display 323. The second storage 322, the second processor 321, the second communication interface 324, and the second display 323 are respectively connected with the second bus 325. The server terminal 330 includes a third bus 334, a third storage 332, a third processor 331, and a third communication interface 333. The third bus 334, the third storage 332, the third processor 331, and the third communication interface 333 are respectively connected with the third bus 334. The first storage 312 stores a first program instruction, and the first processor 311 is configured to execute the first program instruction. The second storage 321 stores a second program instruction, and the second processor 321 is configured to execute the second program instruction. The third storage 332 stores a third program instruction, and the third processor 331 is configured to execute the third program instruction. Data transmissions between any two of the first communication interface 314, the second communication interface 324, and the third communication interface 333 are performed via Bluetooth, infrared, WiFi, Zigbee, or FM.

The first processor 331 executes the first program instruction to control the sensor assembly 313 to detect the objective biological data of the user. The objective biological data includes status information of the user and environment information, where the status information includes a heart rate, a body surface temperature, a movement trajectory, and a velocity of the user, and the environment information includes a location, an atmospheric pressure, and a humidity. The first communication interface 314 transmits the objective biological data.

The second processor 321 executes the second program instruction to control the second communication interface 324 to receive the objective biological data, and obtain the subjective data input by the user. The subjective data includes a height, a weight, an occupation, an interest, and a hobby of the user. The second processor 321 further integrates the objective biological data and the subjective data into a data packet, and controls the second communication interface 324 to transmit the data packet.

The third processor 331 executes the third program instruction to control the third communication interface 333 to receive the data packet, determines the personal information of the user basing on the data packet, and search the social information which matches the personal information in the network social information database. The third communication interface feeds the social information to the intelligent terminal 320. The social information is displayed through the second display 323 of the intelligent terminal 320.

Optionally, the mobile electronic device is a bracelet.

In summary, the social information search system of the present disclosure integrates the usages of the mobile electronic device 310, the intelligent terminal 320, and the server terminal 330, which obtains the objective biological data of the user from the mobile electronic device 10, obtains the subjective data of the user from the intelligent terminal 20, obtains the personal information of the user by analyzing and determining the subjective data and the objective biological data, so as to search the social information which matches the personal information in the network social information database and push the social information to the user, thereby improving the user experience.

The preferred embodiments of the present disclosure are illustrated as above with reference to the drawings, which are not intended to limit the scope of the present disclosure. Any modification, equivalent, and improvement made by those skilled in the art without departing from the scope and spirit of the disclosure should be within the scope of the protection of the present disclosure.

## Claims

1. A social information search system comprising:
a mobile electronic device worn on a user, and comprising a first bus as well as a sensor assembly, a first storage, a first processor, and a first communication interface respectively connected with the first bus;
an intelligent terminal comprising a second bus as well as a second storage, a second processor, a second communication interface, and a second display respectively connected with the second bus; and
a server terminal comprising a third bus as well as a third storage, a third processor, and a third communication interface respectively connected with the third bus;
wherein, the first storage stores a first program instruction, the first processor is configured to execute the first program instruction, the second storage stores a second program instruction, the second processor is configured to execute the second program instruction, the third storage stores a third program instruction , the third processor is configured to execute the third program instruction, a data transmission between any two of the first communication interface, the second communication interface, and the third communication interface is performed via Bluetooth, infrared, WiFi, Zigbee, or FM;
the first processor executes the first program instruction to control the sensor assembly to detect objective biological data of the user, the objective biological data comprising status information of the user and environment information, the status information comprises a heart rate, a body surface temperature, a movement trajectory, and a velocity of the user, the environment information comprises a location, an atmospheric pressure, and a humidity; the first communication interface transmits the objective biological data;
the second processor executes the second program instruction to control the second communication interface to receive the objective biological data, obtain subjective data input by the user, integrate the objective biological data and the subjective data into a data packet, and control the second communication interface to transmit the data packet;
the third processor executes the third program instruction to control the third communication interface to receive the data packet, determines personal information of the user basing on the data packet, and search social information matching the personal information in a network social information database; the third communication interface feeds the social information to the intelligent terminal.

2. The system of claim 1, wherein the subjective data comprises a height, a weight, an occupation, an interest, and a hobby of the user.

3. The system of claim 1, wherein the mobile electronic device is a bracelet.

4. A social information search system, comprising:
a mobile electronic device for wearing by a user, comprising:
an objective data recording module configured to detect objective biological data of the user; and
a first communication unit configured to transmit the objective biological data;
an intelligent terminal, comprising:
a second communication unit configured to receive the objective biological data;
a subjective data recording module configured to obtain subjective data input by the user; and
a first processing chip configured to integrate the subjective data and the objective biological data into a data packet;
the second communication unit is further configured to transmit the data packet;
a server terminal comprising:
a third communication unit configured to receive the data packet; and
a second processing chip configured to determine personal information of the user based on the data packet, and search social information matching the personal information in a network social information database;
wherein the third communication unit is further configured to feed the social information to the intelligent terminal.

5. The system of claim 4, wherein the objective biological data comprises status information of the user and environment information, the status information comprises a heart rate, a body surface temperature, a movement trajectory, and a velocity of the user, the environment information comprises a location, an atmospheric pressure, and a humidity.

6. The system of claim 5, wherein the subjective data comprises a height, a weight, an occupation, an interest, and a hobby of the user.

7. The system of claim 4, wherein a data transmission between any two of the first communication unit, the second communication unit, and the third communication unit is performed via Bluetooth, infrared, WiFi, Zigbee, or FM.

8. The system of claim 4, wherein the mobile electronic device is a bracelet.

9. A social information search method, comprising:
receiving data packet comprising subjective data and objective biological data of a user from an intelligent terminal by a third communication unit;
determining personal information of the user based on the subjective data and the objective biological data and search social information matching the personal information in a network social information database by a second processing chip; and
feeding the social information to the intelligent terminal by the third communication unit.

10. The method of claim 9, wherein the objective biological data comprises status information of the user and environment information, the status information comprises a heart rate, a body surface temperature, a movement trajectory, and a velocity of the user, the environment information comprises a location, an atmospheric pressure, and a humidity.

11. The method of claim 10, wherein the subjective data comprises a height, a weight, an occupation, an interest, and a hobby of the user.

12. The method of claim 9, wherein the receiving the data packet comprising the subjective data of the user and the objective biological data from the intelligent terminal by the third communication unit comprises: receiving the data packet from the intelligent terminal by the third communication unit via Bluetooth, infrared, WiFi, Zigbee, or FM.

13. The method of claim 9, wherein before the receiving the data packet comprising the subjective data of the user and the objective biological data from the intelligent terminal by the third communication unit comprises:
receiving the subjective data of the user from the mobile electronic device by the intelligent terminal.
